# EUROPEAN PATENT SPECIFICATION

(11) **EP 4 186 355 B1**
(45) Date of publication and mention of the grant of the patent: **23.04.2025**
(21) Application number: 21210898.9
(22) Date of filing: 26.11.2021
(51) Int. Cl.: A01G 25/16, G01N 33/00, G01N 33/24

(54) **TELEMETER STATION**
FERNMESSSTATION
STATION DE TÉLÉMÉTRIE

(43) Date of publication of application: 31.05.2023
(73) Proprietor: Neuropublic Anonimi Etaireia Pliroforikis kai Epikoinonion, 18545 Piraeus Attika (GR)
(72) Inventor: Kidonakis, Ioannis, 11526 Athens (GR); Dimou-Sakellariou, Stergios, 15669 Papagou (GR); Moschos, Anastasios, 13671 Acharnai (GR); Chatzipapadopoulos, Fotis, 15349 Anthoussa (GR)
(74) Representative: Kouzelis, Dimitrios

(56) References cited:
- EP-A1- 3 045 844
- AU-A4- 2021 102 664
- KR-B1- 101 506 320
- PT-A- 116 205
- UNKNOWN: "Leaf Wetness Sensor", 10 March 2019 (2019-03-10), pages 1 - 4, XP055913651, Retrieved from the Internet <URL:https://cdn.shopify.com/s/files/1/0515/5992/3873/files/07395-110_IM.pdf> [retrieved on 20220420]
- UNKNOWN: "Precision Weather Instruments", 1 January 2011 (2011-01-01), pages 1 - 32, XP093068062, Retrieved from the Internet <URL:https://www.skystef.be/Davis.pdf> [retrieved on 20230727]
- UNKNOWN: "Vantage Pro 2 Installation Diagram", 31 January 2017 (2017-01-31), pages 1 - 1, XP093068068, Retrieved from the Internet <URL:https://cdn.shopify.com/s/files/1/0515/5992/3873/files/07396_007_ID_6152.pdf> [retrieved on 20230727]
- UNKNOWN: "Leaf Wetness Sensor Specifications Sheet", 11 November 2011 (2011-11-11), pages 1 - 1, XP093068099, Retrieved from the Internet <URL:https://cdn.shopify.com/s/files/1/0515/5992/3873/files/6420_SS.pdf> [retrieved on 20230727]

## Description

### FIELD

The present disclosure relates to a telemeter station for collecting data, in particular agricultural data related to the atmosphere, soil and foliage of the crops.

### BACKGROUND

Telemeter stations are commonly known to be designed to for the in-situ collection of measurements or other data, being capable of transmitting to a remote location important parameter that assist a user to ensure proper monitoring of a crop growth. In detail, such telemeter stations are wireless weather stations that play a significant role for preventing diseases of the plants, and in general of the crop, as well as for obtaining precision of the irrigation process. Such parameters comprise for example, air temperature, air and leaf humidity, leaf moisture etc. With the provision of such parameters, a precise calculation of the crop's water requirements in terms of timing and quantity is succeeded while the risk assessment for any plant disease is more easily carried out since a user can monitor the vitality of the plants of the crop in a continuous manner, thus significantly mitigating the risk of low-quality crop and enhancing therefore the overall production.

AU 2021 102 664 A4 discloses a telemeter station of the related art.

Conventional telemeter stations normally comprise smart devices having integrated sensors and processors for collecting, filtering and delivering the data that is acquired from the environment and from the crop. However, a common drawback of such telemeter stations is their complexity since they need to comprise a plurality of different sensors and processors so as to ensure measurement accuracy. In addition, although conventional telemeter stations provide the capability of remotely collecting environmental data, they lack the provision of remote commanding, such as remote command of irrigation or activation of any protective mechanism so as to protect the site from any severe and sudden weather conditions.

Further, although such telemeter stations can collect environmental data from the soil or the atmosphere, thereby creating a relevant site profile, they cannot guarantee high accuracy of the measurements since they normally rely their outcome on a single source, meaning that a suitable sensor may either be on a single plant or on the telemeter station itself, thereby being unable to ensure that the provided information is reliable for the entire crop. The positioning therefore of the various components of the telemeter station is significant for the reliability of the system. Last, the information that is provided by the conventional telemeter stations is very often delivered to a distant user as numerical values or complex visuals, thereby making it really challenging for a non-specialized user to understand the data and plan his actions accordingly.

It is thus an object of the present disclosure to overcome the aforementioned drawbacks. The described telemeter station is designed to be to collect accurate data of the atmosphere, soil and of the plants of the crop. The herein disclosed station and its relevant components are installed in specific positions and with certain configuration and arrangement such that it ensures reliability of the measured data for the entire site/crop as well as easiness to use for a distant user.

### SUMMARY

According to the invention, a telemeter station for collecting data is provided that comprises a main unit enclosing an electronic subassembly that comprises a processing unit, an atmospheric sensor connected to the processing unit for recording atmospheric data, an elongated rod that is mounted on a ground surface, at least two leaf sensors connected to the processing unit for measuring predefined parameters wherein the first leaf sensor is mounted on the elongated rod and the second leaf sensor is located on a remote position, different from the elongated rod. The main unit is made of UV reinforced polypropylene and comprises a pressure balancing valve with a waterproof breathable membrane.

According to aspects of the disclosure, the at least two leaf sensors comprise a metal support, optionally made of aluminum, and a grid surface, wherein an angle that is formed between the metal support and the grid surface is between 30 and 60 degrees, optionally 45 degrees.

According to aspects of the present disclosure the first leaf sensor the grid is facing towards the north orientation and for the second leaf sensor the grid is facing toward the south orientation.

According to aspects of the present disclosure, the second leaf sensor is mounted on a plant or tree within a crop.

According to aspects of the present disclosure, the telemeter station comprises a soil sensor connected to the processing unit.

According to aspects of the present disclosure, the telemeter station comprises a photovoltaic cell that is connected to the processing unit.

According to aspects of the present disclosure, the atmospheric sensor comprises an anti-theft module.

According to aspects of the present disclosure, the atmospheric sensor, the photovoltaic cell, the soil sensor and the at least two leaf sensors are connected to the processing unit via waterproof plugs.

According to aspects of the disclosure, the remote position of the second leaf sensor is of about 6-10m distance from the elongated rod.

According to aspects of the present disclosure, the predefined parameters measured by the at least two leaf sensors comprise atmospheric leaf temperature, atmospheric leaf humidity and leaf moisture.

According to aspects of the disclosure, the at least two leaf sensors comprise a polyurethane resin molding.

According to other aspects the main unit comprises a moisture protection element, preferably silica gel in the form of an envelope package.

In other aspects of the disclosure, the use of the telemeter station is provided in agricultural production.

In other aspects, a method of remotely collecting environmental data is provided, comprising the steps of providing a telemeter station,
obtaining data by an atmospheric sensor, obtaining data by at least two leaf sensors, Processing the data by the atmospheric sensor and the at least two leaf sensors, share the processed data with another distant device or a cloud server, deliver the processed data, optionally visualize, to a user in a distant location.

Dependent embodiments of the aforementioned aspects are given in the dependent claims and explained in the following description, to which the reader should now refer.

### BRIEF DESCRIPTION OF THE DRAWINGS

Aspects of an embodiment will be described in reference to the drawings, where like numerals reflect like elements:
Figure 1 shows an overview of the telemeter station according to the present inventive concept.
Figures 2A-2C show the two leaf sensors according to the present inventive concept
Figure 3 shows an overview of the soil sensor according to the present inventive concept
Figure 4 shows an overview of the photovoltaic cell according to the present inventive concept
Figure 5 shows an overview of the main unit according to the present inventive concept.

### DETAILED DESCRIPTION

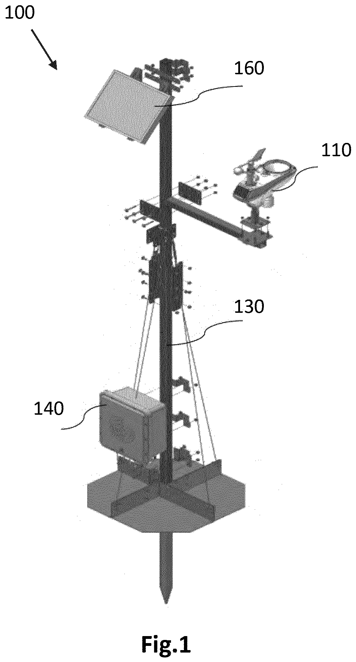

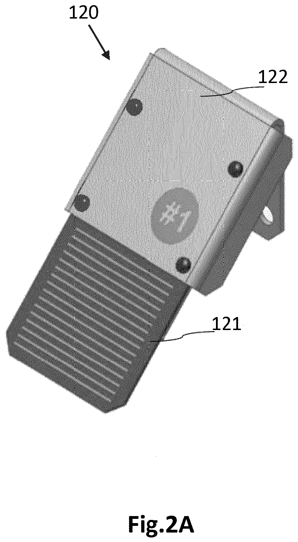

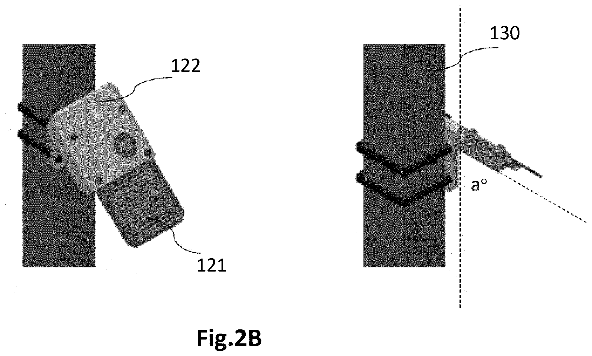

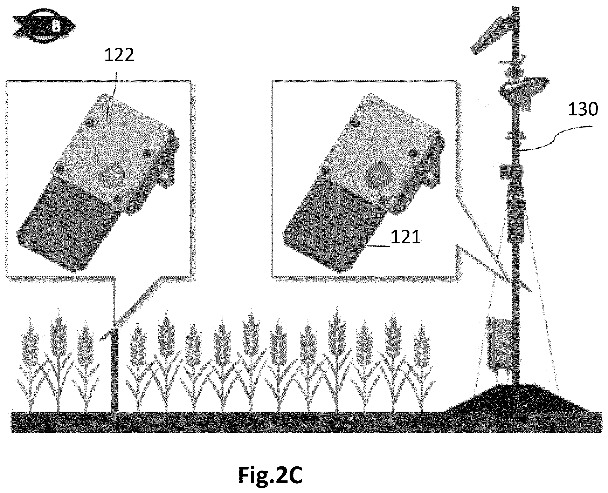

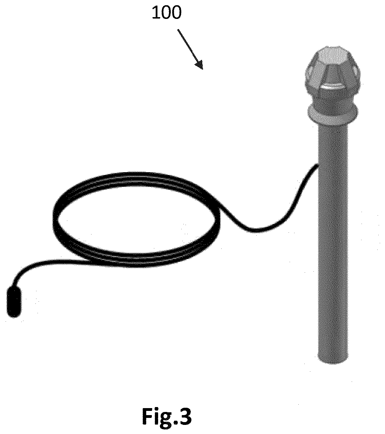

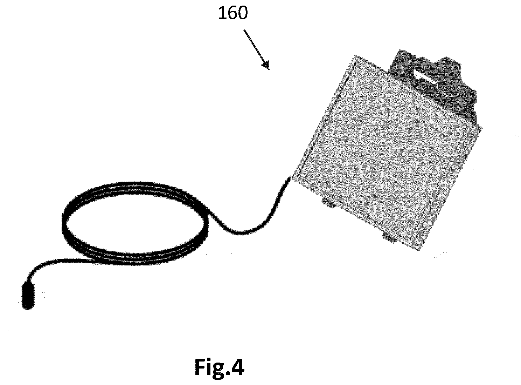

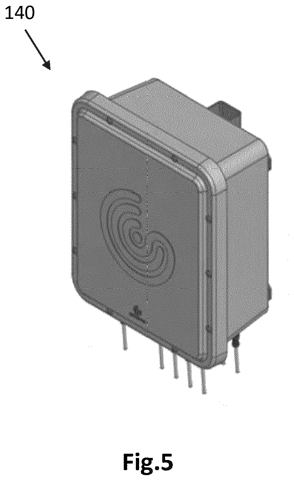

An embodiment of the telemeter station according to aspects of the disclosure will now be described with reference to Figures 1-5. Although the telemeter station is described with reference to specific examples, it should be understood that modifications and changes may be made to these examples without going beyond the general scope as defined by the claims. In particular, individual characteristics of the various embodiments shown and/or mentioned herein may be combined in additional embodiments. Consequently, the description and the drawings should be considered in a sense that is illustrative rather than restrictive. The Figures, which are not necessarily to scale, depict illustrative aspects and are not intended to limit the scope of the disclosure. The illustrative aspects depicted are intended only as exemplary.

The term "exemplary" is used in the sense of "example," rather than "ideal." While aspects of the disclosure are amenable to various modifications and alternative forms, specifics thereof have been shown by way of example in the drawings and will be described in detail. It should be understood, however, that the intention is not to limit aspects of the disclosure to only the described specific embodiment(s). On the contrary, the intention of this disclosure is to cover all modifications, equivalents, and alternatives falling within the scope of the disclosure.

Various materials, methods of construction and methods of fastening will be discussed in the context of the disclosed embodiment(s). Those skilled in the art will recognize known substitutes for the materials, construction methods, and fastening methods, all of which are contemplated as compatible with the disclosed embodiment(s) and are intended to be encompassed by the appended claims.

As used in this disclosure and the appended claims, the singular forms "a," "an," and "the" include plural referents unless the content clearly dictates otherwise. As used in this disclosure and the appended claims, the term "or" is generally employed in its sense including "and/or" unless the content clearly dictates otherwise.

Throughout the description, including the claims, the terms "comprising a," "including a," and "having a" should be understood as being synonymous with "comprising one or more," "including one or more," and "having one or more" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially," "approximately," and "generally" should be understood to mean falling within such accepted tolerances.

When an element or feature is referred to herein as being "on," "engaged to," "connected to," or "coupled to" another element or feature, it may be directly on, engaged, connected, or coupled to the other element or feature, or intervening elements or features may be present. In contrast, when an element or feature is referred to as being "directly on," "directly engaged to," "directly connected to," or "directly coupled to" another element or feature, there may be no intervening elements or features present. Other words used to describe the relationship between elements or features should be interpreted in a like fashion (e.g., "between" versus "directly between," "adjacent" versus "directly adjacent," etc.).

Spatially relative terms, such as "top," "bottom," "middle," "inner," "outer," "beneath," "below," "lower," "above," "upper," and the like, may be used herein for ease of description to describe one element or feature's relationship to another element(s) or feature(s) as illustrated in the drawings. Spatially relative terms may be intended to encompass different orientations of a device in use or operation in addition to the orientation depicted in the drawings. For example, if the device in the drawings is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

Although the terms "first," "second," etc. may be used herein to describe various elements, components, regions, layers, sections, and/or parameters, these elements, components, regions, layers, sections, and/or parameters should not be limited by these terms. These terms are only used to distinguish one element, component, region, layer, or section from another region, layer, or section. Thus, a first element, component, region, layer, or section discussed herein could be termed a second element, component, region, layer, or section without departing from the teachings of the present inventive subject matter.

Figure 1 illustrates a telemeter station 100 for collecting data according to a first embodiment of the invention, comprising a main unit 140 enclosing an electronic subassembly that includes a processing unit, an atmospheric sensor 110 connected to the processing unit, an elongated rod 130 that is mounted on a ground surface, at least two leaf sensors 120 connected to the processing unit for measuring predefined parameters wherein the first leaf sensor is mounted on the elongated rod 130 and the second leaf sensor is located on a remote position, different from the elongated rod.

The data that can be collected by telemeter station 100 may come from the atmosphere, the soil and from the foliage of a crop thereby making the telemeter station 100 perfectly suitable and effective to be used in agricultural production. In detail, such telemeter stations are useful for effective crop management that may include accurate crop damage assessment following severe weather conditions as well as studies on foliage microclimate and meteorological data correlation. Such telemeter stations are installed in specific locations, for example, within a crop, that locations being defined from satellite data so as the data that is collected to be representative for the entire area. The collected data are transmitted to a remote location, thereby providing a user the opportunity to have a reliable and indicative overview of the crop and consequently to alarm him for any corrective actions that may be required, such as irrigation or related to pests and diseases of the plants.

In examples, the processing unit is part of an electronic subassembly that is enclosed within the main unit 140 which has the shape of a box. The processing unit is responsible for the collection, processing, storage and delivery of the recorded data. The hardware of the processing unit is designed such that uninterrupted operation is ensured, since that kind of reliability is highly desired due to the exposure of the system in harsh and severe weather conditions. Further, the firmware of the processing unit ensures that every function of the telemeter stations is properly operated and thereby controlled. In addition, the applicable firmware is designed to collect the data from all the sensors of the telemeter station as will be described in that specification later in more detail (atmospheric sensor 110, leaf sensors etc.), at predefined time intervals, for example less than 10 seconds), process them thereof so as to extract the appropriate data and consequently send the data to the remote user on a frequent basis which may be preferably 6 times per hour, every ten minutes. Additionally, the firmware is designed such that it recognizes the any incorrect data that was sent to the remote user, so as to replace them (with the aid of automated algorithms) and re-send the correct data accordingly. Further, the firmware of the processing unit may also be designed to execute the remote action commands from the remote user. In detail, the firmware recognizes the status of the actuating means of, for example the irrigation, applies the command by the remote use, for example "start irrigation", monitors the entire operation during its duration and indicates to the remote user whether the command has been successfully completed. The processing unit further comprises a battery that is responsible for the power of the entire telemeter station 100.

In examples, the processing unit comprises a 16-bit processor, capable of processing speeds up to 48 MHz with an internal memory of at least 256 Kbytes that is connected to an external memory of at least 512 Mbit. Such configuration allows the processing unit to operate with low level consumption needs that may be less than 1mA. Additionally, the electronic subassembly may comprise at least 8 channels of analog data input with a resolution of about 12 bits, thereby providing an increased capability of digital signal processing which can be translated in local data storage of complete and continuous recoding of measurement packages for a period that may exceed the 18 months. Further, the transmission of the collected data to the remote location may be done through known telecommunication modules, such as but not limited to GSM networks, that are connected to the processing unit through a built in SIM card, which has the ability to select the optimum provider, being independent of the location of the installation of the telemeter station 100.

Additionally, or alternatively, the processing unit may be designed such that a user in a remote location, different from that where the telemeter station 100 is installed, can carry out commanding actions, for example to start and stop irrigation. In that example, and to ensure precise irrigation and unnecessary waste of water, the telemeter station 100 may also comprise a hydrometer to accurately measure the irrigated water. The aforementioned components of the electronic subassembly (processing unit, telecommunication boards, input/output boards, etc..) may be covered by casting polyurethane resin with a thickness of at least 3mm, thereby ensuring that the sensitive electronics of the electronic subassembly are adequately protected both with a cost effective as well as time resistant manner. In other examples, the main unit 140 may also comprise a magnetic manual restart mechanism which should be located on the external surface of the main unit 140. Such mechanism allows the optional restart of the telemeter station 100 without the need to interfere to the internal components of the main unit 140 and consequently without putting at risk any undesired damage to the sensitive components of the electronic subassembly. Conventional main units of telemeter stations utilize button or switches so as to restart their systems. However, these means have a significant cost since they have to be protected from the external conditions, such as the moisture. In addition, the inside part of the main unit 140 may be interrupted since a mounting hole should be drilled on the main unit 140 so as to place the switch inside the main unit 140. On the contrary, providing magnetic contacts provides the advantage of restarting the system externally, thus ensuring that the main unit 140 remains waterproof, while inside the main unit 140 a sound of suitable volume is generated so as to inform the user through audio messages about the status of the station. In detail, within the main unit 140 there may be located a reed magnetic switch. The reed magnetic switch may be activated by another magnet that can be placed on the external surface of the main unit 140. When activated, the reed magnetic switch initiates a reboot sequence for the telemeter station 100. During that reboot sequence, a relatively long, single tone (beep) sound is generated from the inside part of the main unit 140. When the reboot is successful, at least two and preferably two single tones are generated and therefore indicate that the telemeter station is successfully connected to a remote server. From that point, when a data-package is transmitted successfully, a single short beep is generated.

According to the first embodiment of the invention, the main unit 140 is made of UV reinforced polypropylene and comprises a pressure balancing valve with a waterproof breathable membrane. The material of the main unit 140 offers resistance to sunlight and in general to severe weather conditions while it has a sufficient elasticity to seal efficiently the main unit 140 and withstand any impact. The main unit 140 which preferably has a rectangular box shape, may comprise a lid and a body. The lid may have a groove for receiving an elastic cord, said cord being made of EPDM material, which acts as a gasket to seal the box from any water inflow. To further improve this seal, the body of the main unit 140 comprises a protruding rim, cooperating with the groove, configured to increase the pressure on the gasket within the groove. Such protruding rim may be of about 15-20mm. Additionally, to eliminate the effects of moisture suction from the environment due to the vacuum created by the temperature difference between the environment outside and inside the box, a pressure balancing valve with a waterproof breathable membrane is used. The pressure valve balances any pressure differences inside the box, allowing a small air flow, but on the same time preventing the ingress of moisture through the waterproof breathable membrane thus maintaining the waterproofing of the main unit 140. In other examples, the bottom portion of the main unit 140 may comprise a rim of sufficient height that protect the plugs from any undesired water penetration. Such rim may have a height of about 4-8mm, most preferably 6mm, thereby creating a barrier around the exposed plugs, resulting therefore in the mitigation of any risk of the plugs coming in direct contact with rainwater.

In other examples, the main unit 140 comprises a moisture protection element. In examples, the element may be silica gel in the form of an envelope package, inside the main unit 140. The silica gel, which may be of about 100gr, provides the advantage of absorbing the moisture from the inside part of the main unit 140 that may have been trapped during the production of the main unit. In addition, due to the fluctuations of the ambient conditions, some moisture may enter the main unit through ventilation ports that the main unit 140 may comprise. In that case, the silica gel also act as trapping mechanism for the moisture, thereby ensuring proper operation for the main unit and its sub-components.

The telemeter station 100 according to aspects of the present disclosure comprises an atmospheric sensor 110 connected to the processing unit for recording atmospheric data. The atmospheric sensor 110 is used so as to record and collecting of important environmental data that are critical for the monitoring and the overall development of, for example, a crop. The parameters that are recorded by such sensor may comprise atmospheric temperature, humidity and pressure, as well as wind speed and direction, UV power, Solar radiation power and rain precipitation. The connection of the atmospheric sensor 110 to the processing unit may be achieved through waterproof plugs (IP68 certification) that provide increased level of resistance to corrosive conditions that may be present on the site. In examples, the parts of the atmospheric sensor 110 that are exposed to the environment, such as humidity and temperature sensors, are covered and thereby protected, by a breathable membrane, while the electronic components of the atmospheric sensor 110 comprise a polyurethane resin molding that ensure protection against corrosive conditions. In other examples, the atmospheric sensor 110 comprises an anti-theft system which is configured to detect any undesired action during its operation and notifies the remote user through an alarm signal.

Additionally, the telemeter station 100 according to the present disclosure comprises an elongated rod 130 that is mounted on a ground surface. That elongated rod 130, which has the shape of a mast, is used as a support system of the telemeter station 100. In detail, it facilitates the installation of the rest of the components of the telemeter station 100 without hindering the other activities of, for example, the agricultural work. Such elongated rod 130 is removably mounted on the ground and provides the desired space so as to fit all the components of the telemeter station 100 that are required for the proper operation of the system. The elongated rod 130 requires a minor area for installation, less than 0.5 m2 and may have a height between 1-6m, depending on customized needs, thereby allowing the installation independently of the selected area.

In addition, the telemeter station 100 according to the present disclosure comprises at least two leaf sensors connected to the processing unit for measuring predefined parameters wherein the first leaf sensor is mounted on the elongated rod 130 and the second leaf sensor is located on a remote position, different from the elongated rod 130. The leaf sensors are designed such that the provide measurements of high precision for example on atmospheric leaf temperature, leaf humidity and leaf moisture. In that way, a user is ensured about the current condition of the plants on a crop. In examples, each of the leaf sensors comprise only a single sensor for measuring leaf humidity, temperature and moisture, thereby providing to a user a holistic view of the state of the atmosphere in the foliage of a plant with a unique sensor for a plurality of parameters. The leaf sensors are located in different positions; the first is mounted on the elongated rod 130 and the second in a remote position, different from the elongated rod 130. The location of the second leaf sensor may preferably be in the vicinity or even on a plant of a crop, which may be of about 6-10m from the elongated rod 130. The reliability of the measurements is thus enhanced, and the overall condition of a crop is optimized since there are at least two sources of generating data. In that way, and especially for example in a large crop where not all the plants are on equal distance from the telemeter station 100, a user is provided with data on the plants that are representative for the crop in its entirety.

In other embodiments, the at least two leaf sensors comprise a metal support and a grid surface wherein an angle that is formed between the metal support is between 30 and 60 degrees and more specifically of about 45 degrees. It has been proven that such configuration is significantly effective in minimizing water stagnation while at the same time increases measurement range. Further, an inclination of about 45 degrees has been found to be optimal for measuring the leaf wetness phenomenon. The metal support may be directly mounted on the elongated rod 130 via any suitable means, such as but not limited to tie wraps, while its material may be aluminum which provides optimal heat dissipation while at the same time is not severely influenced by the local microclimate phenomena. In other examples, the grid surface of the first leaf sensor that is mounted on the elongated rod 130 is facing towards the north orientation and the grid surface of the second leaf sensor is facing towards the south orientation. This arrangement provides the advantage of further improving the precision of the collected data. By selecting both north facing and south facing leaf sensors it is ensured that the data acquired is precise and accurate since the measurements are provided in both extremities of wetness conditions. In other examples, the at least two leaf sensors are connected to the processing unit via waterproof plugs that are resistant to corrosive conditions that may prevail in the site. In other examples, the electronics of each leaf sensor of the at least two sensors are protected by polyurethane resin molding while the grid surface that comprises the atmospheric humidity/temperature sensor may be protected by a breathable membrane so as the entire leaf sensor is protected by the variable weather conditions as well as from any chemical spraying that may occur in a crop.

According to other aspects of the disclosure, the telemeter station 100 may comprise a soil sensor 150 that is connected to the processing unit. The soil sensor 150 is preferably mounted on the ground, reaching to a depth that may vary between 10 - 70cm below the ground surface. The soil sensor 150 is configured to measure or estimate soil parameters, for example soil moisture and water content in the soil, thereby contributing to the formulation of the overall condition of a crop.

In other examples, the telemeter station 100 comprises a photovoltaic cell 160 that is connected to the processing unit. The photovoltaic cell 160 may preferably me mounted on the elongated rod 130 via any suitable means and provides the telemeter station 100 an absolute energy autonomy because of the solar radiation exploitation. The installation of the photovoltaic cell 160 and especially the mounting angle, may vary within the year, due to the geographical data so as to optimize the intake of the solar radiation, especially during the winter months. Such mounting angle may be 30 and 60 degrees relative to a horizontal plane as defined by the photovoltaic cell 160. The photovoltaic cell 160 may be equipped with power of at least 10 Wp so as uninterrupted charging of the telemeter station 100 is ensured even with low solar radiation conditions.

According to other aspects of the disclosure, a method of remotely collecting environmental data is provided that comprises the steps of providing a telemeter station 100, obtaining data by an atmospheric sensor 110, obtaining data by at least two leaf sensors 120, processing the data by the atmospheric sensor 110 and the at least two leaf sensors 120, sharing the processed data with another distant device or a cloud server, deliver the processed data, optionally visualize, to a user in a distant location.

According to other aspects of the disclosure, use of a telemeter station is provided in agricultural production.

It should be noted that the above embodiments are only for illustrating and not limiting the technical solutions of the present invention. Although the present invention has been described in detail with reference to the above embodiments, those skilled in the art should understand that any modifications or equivalent substitutions of the present invention are intended to be included within the scope of the appended claims.

Although the present disclosure herein has been described with reference to particular embodiments, it is to be understood that these embodiments are merely illustrative of the principles and applications of the present disclosure.

It is intended that the specification and examples be considered as exemplary only, with a true scope of the invention as defined by the following claims.

## Claims

1. A telemeter station (100) for collecting data comprising:
- a main unit (140) enclosing an electronic subassembly, the electronic subassembly comprising a processing unit,
- an atmospheric sensor (110) connected to the processing unit for recording atmospheric data,
- an elongated rod (130) that is mounted on a ground surface,
- at least two leaf sensors (120) connected to the processing unit for measuring predefined parameters,
wherein the first leaf sensor (120) is mounted on the elongated rod (130) and the second leaf sensor (120) is located on a remote position, different from the elongated rod (130), **characterised in that** the main unit (140) is made of UV reinforced polypropylene and further comprises a pressure balancing valve with a waterproof breathable membrane.

2. The telemeter station (100) according to claim 1, wherein the at least two leaf sensors (120) comprise a metal support (122), optionally made of aluminum, and a grid surface (121), wherein an angle that is formed between the metal support (122) and the grid surface (121) is between 30 and 60 degrees, preferably 45 degrees.

3. The telemeter station (100) according to any of the preceding claims comprising a soil sensor (150) connected to the processing unit.

4. The telemeter station (100) according to any of the preceding claims comprising a photovoltaic cell (160) that is connected to the processing unit.

5. The telemeter station (100) according to any of the preceding claims, wherein the atmospheric sensor (110) comprises an anti-theft module.

6. The telemeter station according to claim 4, wherein the atmospheric sensor (110), the photovoltaic cell 160, the soil sensor 150 and the at least two leaf sensors (120) are connected to the processing unit via waterproof plugs.

7. The telemeter station (100) according to any of the preceding claims, wherein the predefined parameters measured by the at least two leaf sensors (120) comprise atmospheric leaf temperature, atmospheric leaf humidity and leaf moisture.

8. The telemeter station (100) according to any of the preceding claims, wherein the at least two leaf sensors (120) comprise a polyurethane resin molding.

9. The telemeter station (100) according to any of the preceding claims, wherein the main unit (140) comprises a moisture protection element, preferably silica gel in the form of an envelope package.

10. Use of the telemeter station (100) according to any of the preceding claims in agricultural production.

11. A method of remotely collecting environmental data comprising the steps of:
- Providing a telemeter station according to claims 1-9
- Obtaining data by an atmospheric sensor (110)
- Obtaining data by at least two leaf sensors (120)
- Processing the data by the atmospheric sensor (110) and the at least two leaf sensors (120)
- Sharing the processed data with another distant device or a cloud server
- Deliver the processed data, optionally visualize, to a user in a distant location

12. The method according to claim 11, wherein for the first leaf sensor (120) the grid surface (122) is facing towards the north orientation and for the second leaf sensor (120) the grid surface (122) is facing toward the south orientation.

13. The method according to claim 11 or 12, wherein the second leaf sensor (120) is mounted on a plant or tree within a crop.

14. The method according to any of claims 11 to 13, wherein the remote position of the second leaf sensor (120) is of about 6-10m distance from the elongated rod (130).

## Patentansprüche

1. Fernmessstation (100) zum Erfassen von Daten, umfassend:
- eine Haupteinheit (140), die eine elektronische Unterbaugruppe umschließt, wobei die elektronische Unterbaugruppe eine Verarbeitungseinheit umfasst,
- einen Atmosphärensensor (110), der mit der Verarbeitungseinheit zum Aufzeichnen von atmosphärischen Daten verbunden ist,
- eine längliche Stange (130), die auf einer Bodenfläche montiert ist,
- mindestens zwei Blattsensoren (120), die mit der Verarbeitungseinheit zum Messen von vordefinierten Parametern verbunden sind,
wobei der erste Blattsensor (120) an der länglichen Stange (130) angebracht ist und der zweite Blattsensor (120) an einer entfernten Position, die unterschiedlich von der länglichen Stange (130) ist, angeordnet ist, **dadurch gekennzeichnet, dass**
die Haupteinheit (140) aus UV-verstärktem Polypropylen gefertigt ist und ferner ein Druckausgleichsventil mit einer wasserdichten, atmungsaktiven Membran umfasst.

2. Fernmessstation (100) nach Anspruch 1, wobei die mindestens zwei Blattsensoren (120) einen Metallträger (122), optional aus Aluminium, und eine Gitterfläche (121) umfassen, wobei ein Winkel, der zwischen dem Metallträger (122) und der Gitterfläche (121) gebildet ist, zwischen 30 und 60 Grad, vorzugsweise 45 Grad, beträgt.

3. Fernmessstation (100) nach einem der vorhergehenden Ansprüche, umfassend einen Bodensensor (150), der mit der Verarbeitungseinheit verbunden ist.

4. Fernmessstation (100) nach einem der vorhergehenden Ansprüche, umfassend eine photovoltaische Zelle (160), die mit der Verarbeitungseinheit verbunden ist.

5. Fernmessstation (100) nach einem der vorhergehenden Ansprüche, wobei der Atmosphärensensor (110) ein Anti-Diebstahl-Modul umfasst.

6. Fernmessstation nach Anspruch 4, wobei der Atmosphärensensor (110), die photovoltaische Zelle 160, der Bodensensor 150 und die mindestens zwei Blattsensoren (120) über wasserdichte Stecker mit der Verarbeitungseinheit verbunden sind.

7. Fernmessstation (100) nach einem der vorhergehenden Ansprüche, wobei die von den mindestens zwei Blattsensoren (120) gemessenen vordefinierten Parameter die atmosphärische Blatttemperatur, die atmosphärische Blattfeuchte und die Blattnässe umfassen.

8. Fernmessstation (100) nach einem der vorhergehenden Ansprüche, wobei die mindestens zwei Blattsensoren (120) ein Polyurethanharzformteil umfassen.

9. Fernmessstation (100) nach einem der vorhergehenden Ansprüche, wobei die Haupteinheit (140) ein Feuchtigkeitsschutzelement, vorzugsweise Silikagel in Form einer Hüllverpackung, umfasst.

10. Verwendung der Fernmessstation (100) nach einem der vorhergehenden Ansprüche in landwirtschaftlicher Produktion.

11. Verfahren zum Fernerfassen von Umweltdaten, umfassend die folgenden Schritte:
- Bereitstellen einer Fernmessstation nach einem der Ansprüche 1-9
- Erhalten von Daten mittels eines Atmosphärensensors (110)
- Erhalten von Daten mittels mindestens zwei Blattsensoren (120)
- Verarbeiten der Daten von dem Atmosphärensensor (110) und der mindestens zwei Blattsensoren (120)
- Teilen der verarbeiteten Daten mit einem anderen entfernten Gerät oder einem Cloud-Server
- Übermitteln der verarbeiteten Daten, optional Visualisieren, an einen Benutzer an einem entfernten Ort

12. Verfahren nach Anspruch 11, wobei für den ersten Blattsensor (120) die Gitterfläche (122) in Richtung Norden und für den zweiten Blattsensor (120) die Gitterfläche (122) in Richtung Süden ausgerichtet ist.

13. Verfahren nach Anspruch 11 oder 12, wobei der zweite Blattsensor (120) an einer Pflanze oder einem Baum in einem Bestand angebracht ist.

14. Verfahren nach einem der Ansprüche 11 bis 13, wobei die entfernte Position des zweiten Blattsensors (120) etwa 6-10 m von der länglichen Stange (130) beträgt.

## Revendications

1. Une station télémétrique (100) pour la collecte de données comprenant:
- une unité principale (140) renfermant un sous-ensemble électronique, le sous-ensemble électronique comprenant une unité de traitement
- un capteur atmosphérique (110) connecté à l'unité de traitement pour enregistrer les données atmosphériques,
- une tige allongée (130) qui est montée sur une surface au sol,
- au moins deux capteurs de feuilles (120) reliés à l'unité de traitement pour mesurer des paramètres prédéfinis,
desquelles le premier capteur de feuilles (120) est monté sur la tige allongée (130) et le second capteur de feuilles (120) est situé à une position éloignée, différente de la tige allongée (130), **caractérisé par le fait que**
l'unité principale (140) est fabriquée en polypropylène renforcé aux UV et comprend en outre une valve d'équilibrage de pression avec une membrane imperméable et respirante.

2. La station télémétrique (100) selon la revendication 1, dans laquelle les au moins deux capteurs de feuilles (120) comprennent un support métallique (122), éventuellement en aluminium, et une surface quadrillée (121), dans lesquelles un angle formé entre le support métallique (122) et la surface quadrillée (121) est compris entre 30 et 60 degrés, de préférence 45 degrés.

3. La station télémétrique (100) selon l'une quelconque des revendications précédentes comprenant un capteur de sol (150) relié à l'unité de traitement.

4. La station télémétrique (100) selon l'une quelconque des revendications précédentes comprenant une cellule photovoltaïque (160) reliée à l'unité de traitement.

5. La station télémétrique (100) selon l'une quelconque des revendications précédentes, dans laquelle le capteur atmosphérique (110) comprend un module antivol.

6. La station télémétrique selon la revendication 4, dans laquelle le capteur atmosphérique (110), la cellule photovoltaïque (160), le capteur de sol (150) et les au moins deux capteurs de feuilles (120) sont reliés à l'unité de traitement par des prises étanches.

7. La station télémétrique (100) selon l'une quelconque des revendications précédentes, dans laquelle les paramètres prédéfinis mesurés par les au moins deux capteurs de feuilles (120) comprennent la température atmosphérique des feuilles, l'humidité atmosphérique des feuilles et l'humidité des feuilles.

8. La station télémétrique (100) selon l'une quelconque des revendications précédentes, dans laquelle les au moins deux capteurs de feuilles (120) sont constitués d'un moulage en résine polyuréthane.

9. La station télémétrique (100) selon l'une des revendications précédentes, dans laquelle l'unité principale (140) comprend un élément de protection contre l'humidité, de préférence du gel de silice sous forme d'enveloppe.

10. Utilisation de la station télémétrique (100) selon l'une quelconque des revendications précédentes dans la production agricole.

11. Méthode de collecte à distance de données environnementales comprenant les étapes suivantes :
- Fourniture d'une station télémétrique selon les revendications 1 à 9
- Obtention de données par un capteur atmosphérique (110)
- Obtention de données par au moins deux capteurs de feuilles (120)
- Traitement des données par le capteur atmosphérique (110) et les au moins deux capteurs de feuilles (120)
- Partager les données traitées avec un autre dispositif distant ou un serveur en nuage
- Fournir les données traitées, éventuellement les visualiser, à un utilisateur éloigné.

12. Méthode selon la revendication 11, dans laquelle, pour le premier capteur de feuilles (120), la surface de la grille (122) est orientée vers le nord et, pour le second capteur de feuilles (120), la surface de la grille (122) est orientée vers le sud.

13. Méthode selon la revendication 11 ou 12, dans laquelle le second capteur de feuilles (120) est monté sur une plante ou un arbre dans une culture.

14. La méthode selon l'une des revendications 11 à 13, dans laquelle la position éloignée du second capteur de feuilles (120) se situe à une distance d'environ 6 à 10 m de la tige allongée (130).
